# EUROPEAN PATENT SPECIFICATION

(11) **EP 4 030 787 B1**
(45) Date of publication and mention of the grant of the patent: **04.06.2025**
(21) Application number: 21152082.0
(22) Date of filing: 18.01.2021
(51) Int. Cl.: H04W 4/02, H04W 4/029, G06F 21/62, G16H 50/80, G08B 21/22

(54) **METHOD FOR ENABLING CONTACT DETECTION AMONG A PLURALITY OF PERSONS BY MEANS OF DETECTING RELEVANT ENCOUNTERS BETWEEN TWO PERSONS, SYSTEM, EDGE DEVICE AND WEARABLE OR PORTABLE DEVICES, PROGRAM AND COMPUTER-READABLE MEDIUM**
VERFAHREN ZUR ERMÖGLICHUNG DER KONTAKTDETEKTION UNTER EINER VIELZAHL VON PERSONEN MITTELS DETEKTION RELEVANTER BEGEGNUNGEN ZWISCHEN ZWEI PERSONEN, SYSTEM, EDGE-VORRICHTUNG UND WEARABLE- ODER TRAGBARE VORRICHTUNGEN, PROGRAMM UND COMPUTERLESBARES MEDIUM
PROCÉDÉ D'ACTIVATION DE DÉTECTION DE CONTACT PARMI UNE PLURALITÉ DE PERSONNES AU MOYEN DE LA DÉTECTION DE RENCONTRES PERTINENTES ENTRE DEUX PERSONNES, SYSTÈME ET DISPOSITIF DE BORD OU DISPOSITIFS PORTATIFS OU PORTABLES, PROGRAMME ET SUPPORT LISIBLE PAR ORDINATEUR

(43) Date of publication of application: 20.07.2022
(73) Proprietor: Deutsche Telekom AG, 53113 Bonn (DE)
(72) Inventor: ROLLIN, Hannes, 19055 Schwerin (DE); MITEV, Martin, 53113 Bonn (DE)
(74) Representative: Schwöbel, Thilo K.

(56) References cited:
- DE-U1- 202020 103 175
- WILLIAM J BUCHANAN ET AL: "Review and Critical Analysis of Privacy-preserving Infection Tracking and Contact Tracing", ARXIV.ORG, CORNELL UNIVERSITY LIBRARY, 201 OLIN LIBRARY CORNELL UNIVERSITY ITHACA, NY 14853, 10 September 2020 (2020-09-10), XP081760207
- "Comparison of existing pandemic contact tracing systems", vol. ISG - E4P, no. V1.1.1, 17 December 2020 (2020-12-17), pages 1 - 113, XP014396414, Retrieved from the Internet <URL:ftp://docbox.etsi.org/ISG/E4P/Open/e4p-002v111publishedversion_v120internalversion.pdf> [retrieved on 20201217]

## Description

### BACKGROUND

The present invention relates to a method for enabling contact tracing among a plurality of persons by means of detecting relevant encounters between two persons, wherein each of the plurality of persons are wearing or carrying a wearable or portable device.

Furthermore, the present invention relates to a system for enabling contact tracing among a plurality of persons by means of detecting relevant encounters between two persons, wherein each of the plurality of persons are wearing or carrying a wearable or portable device.

Additionally, the present invention relates to an edge device and to wearable or portable devices for enabling contact tracing among a plurality of persons by means of detecting relevant encounters between two persons, wherein each of the plurality of persons are wearing or carrying a wearable or portable device.

Furthermore, the present invention relates to wearable or portable devices for enabling contact tracing among a plurality of persons by means of detecting relevant encounters between two persons, wherein each of the plurality of persons are wearing or carrying a wearable or portable device.

Additionally, the present invention relates to a program and to a computer-readable medium for enabling contact tracing among a plurality of persons by means of detecting relevant encounters between two persons according to the inventive method.

It is conventionally known to realize contact detection and/or contact tracing for a plurality of persons by means of using wearable or portable devices, especially socalled ultra-wideband wearable or portable devices, or ultra-wideband tags. Such wearable or portable devices typically store log data related to the contact situation of the respective or considered wearable or portable device with other wearable or portable devices being or having been in proximity during a considered time interval such as, e.g., a day or a work shift, thereby providing the possibility to either help to avoid the spread of infectious diseases or at least to be able to detect encounters between at least two persons and later on to trace such contacts or encounters.

However, such log data need to be analyzed properly in order to detect the relevant encounters that carry a higher risk of involving a transmission.

State of the art of contact tracing is disclosed in paper "Review and Critical Analysis of Privacypreserving Infection Tracking and Contact Tracing", DE202020103175U U1 and ETSI Draft "ETSI GR E4P-002 V1.1.1- published version-V1.2.0 Comparison of existing pandemic contact tracing systems".

### SUMMARY

An object of the present invention is to provide a technically simple, effective, explainable, and cost-effective solution for enabling contact tracing among a plurality of persons by means of detecting relevant encounters between two persons. A further object of the present invention is to provide a corresponding system, an edge device, wearable or portable devices, and a corresponding program and computer-readable medium.

The object of the present invention is achieved by a method for enabling contact tracing among a plurality of persons by means of detecting relevant encounters between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices,
wherein, in a contact situation of a specific wearable or portable device with a specific further wearable or portable device, the wearable or portable device is able, by means of the wearable or portable device's wireless communication interface and with respect to the further wearable or portable device, to determine repeatedly the distance, at different points in time, towards the further wearable or portable device and to receive the further wearable or portable device's tag identifier, thereby generating and storing log data of the contact situation of the wearable or portable device with the further wearable or portable device,
wherein in order to detect relevant encounters of the wearable or portable device with the further wearable or portable device the method comprises the following steps:
   -- in a first step, an available plurality of relevant distance detection events involving the further wearable or portable device, detected by the wearable or portable device, is determined by means of - among all available distance detection events of the further wearable or portable device, detected by the wearable or portable device - considering or selecting only such distance detection events indicating the further wearable or portable device to be positioned at a distance, from the wearable or portable device, of below a predetermined distance threshold or at most equal to the predetermined distance threshold,
   -- in a second step, regarding the available plurality of relevant distance detection events, involving the further wearable or portable device and detected by the wearable or portable device, one or a plurality of continuous encounter time intervals are determined or generated regarding chronologically supposedly continuous encounters - of at maximum the predetermined distance threshold between the wearable or portable device and the further wearable or portable device -, wherein each continuous encounter time interval corresponds to a certain number of relevant distance detection events, each of whose comprising or being assigned to a respective point in time of the respective distance detection event, the duration of the continuous encounter time interval being defined as or being determined to correspond to the time difference between the most recent and the earliest of these points in time, respectively,
   -- in a third step, based on the one or the plurality of detected or generated continuous encounter time intervals regarding the further wearable or portable device, an encounter duration is determined, the encounter duration corresponding to the sum of the durations of the continuous encounter time intervals,
wherein a relevant encounter is detected in case that the encounter duration exceeds a predetermined duration threshold.

It is thereby advantageously possible according to the present invention to effectively detect the relevant encounters of two wearable or portable devices, and thereby to be able to concentrate on such relevant encounters, possibly leading to significantly reduce the amount of log data to be taken into consideration.

When using contact detecting and/or tracing tags or wearable or portable devices, these tags or wearable or portable devices allow to combine high-precision contact tracing - especially if ultra-wideband, UWB, wearables or wearable devices or tags are used - especially with a decentralized risk calculation on a computing device, often or typically the users' smartphones. Typically, each wearable or portable device or tag has a unique ID, or tag identifier which is broadcast and saved by other wearable or portable devices or tags in the vicinity. The sum or combination of these measurements gives rise to logs (or log data), which are stored on the wearable or portable device or tag and are typically transported to a computing device, often a user's smart phone or other personal digital assistant, or part of the wearable or portable device, where the log data must be processed to detect risk encounters.

The wearable or portable devices might either not be personalized per se (and in this case they can be used and reused by anyone (typically among the group of persons concerned, e.g. the staff of an organization or a differently defined group of persons)), but during one session, only a single user is typically using one wearable device or tag. According to another embodiment of the present invention, the wearable or portable device is able to provide the functionality of both a tag (or wearable device, i.e. primarily directed to contact detection) and of a computing device, i.e. for analyzing the log data.

According to the present invention, normally a certain number of persons is considered such as, especially, the staff of an enterprise or of an organization. It is aimed at enabling contact detection and contact tracing for these persons such that contact tracing is able to be performed individually for each person, and typically performed by each person individually using their personal computing device, especially a smart phone or other personal portable device. However, as the wearable or portable device does not necessarily be a dedicated contact tracking or detecting device but might also be embodied as, e.g., a mobile phone of the person wearing or carrying it, the number of persons is not necessarily strictly limited a priori to, e.g., the staff of an enterprise or of an organization.

During operation, e.g. during a work shift or during a work day, each person is wearing or carrying a wearable or portable device or tag (typically one, but, in principle, it is not excluded to wear more than one tag) of the plurality of wearable or portable devices. To each wearable or portable device is assigned or each wearable or portable device is associated to a tag identifier, i.e. an identifying information such as a serial number or the like. At a specific point in time, each wearable or portable device or each tag is associated to exactly one tag identifier; however, in principle according to the present invention, it is not excluded that one and the same wearable or portable device or tag is able to be associated or assigned (successively) to more than one wearable or portable device, especially in order to still enhance the level of data protection or data privacy, especially by avoiding to create specific pattern, e.g. regarding the usage of certain wearable or portable devices or tags by certain persons or the like. However, in case that more than one tag identifiers are able to be associated or assigned to one and the same wearable or portable device, a mechanism is needed to securely identify or determine such tag identifier without doubt each time the wearable or portable device is used. In the following, the invention is mainly described under the assumption that a tag identifier is fixedly, i.e. unchangeably, associated or assigned to a wearable or portable device.

According to the present invention, the wearable or portable devices, while in operation, more or less continuously, i.e. repeatedly, broadcast the assigned or associated (at this point in time unique) tag identifier (using the wireless communication interface of the wearable or portable devices), i.e. these broadcasts are typically performed, by the wearable or portable devices and under normal conditions, by applying a certain rate of broadcasts (i.e. a number of broadcasts per time unit, such as, typically once per second (i.e. 1 Hz) or the like; usual such broadcast rates might range from about 0,1 Hz (i.e. once every 10 seconds) to about 10 Hz (ten broadcasts per second)); a broadcast typically corresponding to a radio frequency signal being emitted by the respective wearable or portable device applying a certain standardized or usual emission power such that another wearable or portable device, located in proximity, is able to receive such radio frequency signal and derive or determine, from some signal parameter (such as, typically, some signal strength parameter) and/or from some signal content (such as, e.g., time stamp information and/or emission power information) an indication regarding the distance between both wearable or portable devices. Of course, a corresponding broadcast and radio frequency signal reception procedure is symmetric and both are occurring simultaneously. This also means that, in case that more than two wearable or portable devices are present or located at a specific location or within a considered area (of a size or extension smaller than the radio frequency coverage area of each of the wearable or portable devices), there might be many broadcasts and many radio frequency signals to be received and processed, often leading to - in case of a density of wearable or portable devices (and/or in case of a rate of radio frequency signals to be received and/or processed by each one of the wearable or portable devices considered) exceeding a certain density threshold - the wearable or portable devices broadcasting the radio frequency signals less often in such high density conditions, i.e. the above mentioned rate of broadcasts (under normal conditions) might be adaptively reduced in such high density conditions. However, each such broadcast event of a wearable or portable device involves the wearable or portable device transmitting or broadcasting its (at least temporarily) associated or assigned tag identifier (hereinafter also called tag identifier information).

Hence according to the present invention, in a contact situation of a wearable or portable device with a further wearable or portable device (of the plurality of wearable or portable devices), the wearable or portable device is able, by means of the wearable or portable device's wireless communication interface and with respect to the further wearable or portable device, to determine repeatedly the distance, at different points in time, towards the further wearable or portable device and to receive (typically at a plurality of points in time) the wearable or portable device's tag identifier. From these received broadcast pieces of information (that each wearable or portable device is able to receive from the other wearable or portable devices nearby, i.e. within the coverage area of the respective other wearable or portable device), each wearable or portable device generates (and typically stores) log data, this log data corresponding to or representing the contact situation of the wearable or portable device with the further wearable or portable device (in case of only one further wearable or portable device) or with the plurality of further wearable or portable devices (in case of more than one further wearable or portable device nearby). The log data typically comprise all distance detection events (of or measured or detected by the wearable or portable device with respect to any nearby wearable or portable devices whose radio frequency signals are detectable or receivable by the wearable or portable device), i.e. all detections of the wearable or portable device with other wearable or portable devices. For example, each contact or each distance detection event could be represented, as part of the log data by the following quadruple:
<My ID (tag identifier of the wearable or portable device), Other ID (tag identifier of the further wearable or portable device), Timestamp (the point in time of the contact or distance detection event), Distance (the distance measured or determined by the wearable or portable device)>

According to the present invention, the complete log data (of the wearable or portable device) is split into parts concerning only two wearable or portable devices ('binary parts'), i.e. sub-logs that only concern, e.g., the wearable or portable device and the further wearable or portable device (but no other wearable or portable devices); the result being that each such sub-log contains only contacts (or distance detection events from the perspective of one wearable or portable device) with exactly one other wearable or portable device or tag, i.e. within each sub-log, there's just a single, constant "Other ID". In order to further reduce the relevant log data, of this complete set of 'binary' distance detection events (i.e. of the wearable or portable device with the further wearable or portable device), only the relevant distance detection events are considered, i.e. having a sufficiently low distance indication.

According to the present invention, by means of detecting relevant encounters of the wearable or portable device with the further wearable or portable device (or being able to effectively detect the relevant encounters, i.e. only consider or extract the really relevant encounters), it is possible to concentrate on such relevant encounters, possibly leading to significantly reduce the amount of log data to be taken into consideration. According to the present invention, this is possible to be implemented by means of the first step, during which an available plurality of relevant distance detection events involving the further wearable or portable device, detected by the wearable or portable device, is determined by means of - among all available distance detection events of the further wearable or portable device, detected by the wearable or portable device - considering or selecting only such distance detection events indicating the further wearable or portable device to be positioned at a distance, from the wearable or portable device, of below a predetermined distance threshold or at most equal to the predetermined distance threshold. To detect and, afterwards restrict the processing only on such relevant distance detection events reduces the processing efforts and requirements considerable compared to having process the complete log data, sometimes containing tens of thousands of contacts (or distance detection events) with dozens or hundreds of other users (or wearable or portable devices), potential scanning failures, extremely brief close encounters (such as passing each other in a hallway), and extremely long encounters (such as working side by side in an appropriate distance) with potentially high risk or dangerous intimate periods.

In the second step according to the present invention, among the relevant distance detection events, the continuous encounter time intervals are determined or generated: Regarding the available plurality of relevant distance detection events, involving the further wearable or portable device and detected by the wearable or portable device, one or a plurality of continuous encounter time intervals are determined or generated regarding chronologically supposedly continuous encounters (of course of at maximum the predetermined distance threshold between the wearable or portable device and the further wearable or portable device, as otherwise it would have been discarded in the first step), wherein each continuous encounter time interval corresponds to (i.e. aggregates) a certain number of relevant distance detection events, each of whose comprising or being assigned to a respective point in time of the respective distance detection event, the duration of the continuous encounter time interval being defined as or being determined to correspond to the time difference between the most recent and the earliest of these points in time, respectively.

In the third step, based on the one or the plurality of detected or generated continuous encounter time intervals regarding the further wearable or portable device, an encounter duration is determined, the encounter duration corresponding to the sum of the durations of the continuous encounter time intervals,
wherein a relevant encounter is detected in case that the encounter duration exceeds a predetermined duration threshold.

By means of determining or detecting relevant encounters, it is advantageously possible to further reduce the amount of relevant log data or log data to be processed, and thereby to concentrate on such relevant encounters only.

According to the present invention, it is advantageously possible and preferred that, regarding, during the second step, the available plurality of relevant distance detection events, involving the further wearable or portable device and detected by the wearable or portable device, a chronologically continuous encounter is supposed to have occurred and, correspondingly, a continuous encounter time interval is determined or generated in case that the maximum time difference of consecutively detected or logged distance detection events - regarding their respective points in time in consecutive order - is smaller than or equal to a maximum gap duration.

Thereby, it is advantageously possible to provide for an efficient solution that is robust regarding occasional scanning errors or transmission and/or reception failures of broadcast messages between the wearable or portable devices.

According to the present invention, it is furthermore advantageously possible and preferred that the plurality of relevant distance detection events is determined, especially by means of considering all available distance detection events related to the further wearable or portable device, either continuously or at the end of a logging time interval, wherein the logging time interval especially corresponds to, or approximately corresponds to, one of out of the following:
-- one day or 24 hours,
-- one work shift,
-- 16 hours or 12 hours or 10 hours or 9 hours or 8 hours or 6 hours or 4 hours,
-- 3 hours or 2 hours or 1 hour.

Thereby, it is advantageously possible to provide for a flexible data processing adapted to the respective needs or requirements or either the users of the system used. Especially, the available plurality of relevant distance detection events is determined out of the plurality of all available distance detection events. The meaning of available in this respect refers to the point in time when this processing occurs. Typically, in case of comparatively simple tag devices used as wearable or portable devices, such processing is not provided by such tag devices themselves, but, rather, by computing devices to which the respective log data need to be transferred (typically by means of using edge devices to which the respective tag devices are returned after use by a person). Hence, 'available' in this respect corresponds to the point in time after the tags have been returned. In case, however, that the wearable or portable device is provided with sufficient computing functionality (i.e. a sufficiently powerful data processing unit or module as part of the wearable or portable device), this processing might occur within the wearable or portable device itself, and in shorter periods of time; hence, 'available' in this respect corresponds to the point in time of such a processing.

According to the present invention, it is furthermore advantageously possible and preferred that in case that a relevant encounter is detected, a relevant encounter event is generated, wherein especially the relevant encounter event comprises or is related to - especially besides the wearable or portable device's tag identifier and the further wearable or portable device's tag identifier - at least one out of the following:
-- all corresponding continuous encounter time intervals related to the relevant encounter event,
-- the encounter duration, derived from all corresponding continuous encounter time intervals related to the relevant encounter event, as well as the respective earliest as well as most recent points in time of the overall time span of the relevant encounter event,
-- the encounter duration, derived from all corresponding continuous encounter time intervals related to the relevant encounter event, as well as the overall time span of the relevant encounter event and an indicative time stamp information of either the beginning, or the end or the middle of the overall time span of the relevant encounter event,
-- the encounter duration, derived from all corresponding continuous encounter time intervals related to the relevant encounter event.

Thereby, it is advantageously possible to effectively handle the log data generated by the wearable or portable devices.

According to the present invention, it is advantageously furthermore possible and preferred that, as part of the log data generated and stored by the wearable or portable device, each one of the distance detection events, and likewise each one of the relevant distance detection events, detected or generated with regard to the further wearable or portable device, comprises a quadruple of:
-- the wearable or portable device's tag identifier,
-- the further wearable or portable device's tag identifier,
-- a time stamp information indicative of the point in time of the respective distance detection event,
-- a distance information indicative of the detected or measured distance between the wearable or portable device and the further wearable or portable device,
wherein especially the relevant distance detection events are the ones among all available distance detection events of or detected by the wearable or portable device having a distance information below the predetermined distance threshold.

According to the present invention, it is furthermore advantageously possible and preferred that each one of the continuous encounter time intervals, generated from or based on the available distance detection events and the available relevant distance detection events and detected or generated with regard to both the wearable or portable device and the further wearable or portable device, comprises both the wearable or portable device's tag identifier and the further wearable or portable device's tag identifier, as well as:
-- either a time stamp information indicative of the beginning or and end of the respective continuous encounter time interval as well as a duration information of the continuous encounter time interval,
-- or a time stamp information of the beginning of the respective continuous encounter time interval and a time stamp information of the end of the respective continuous encounter time interval.

According to a further preferred embodiment of the present invention, the determination or selection of the relevant distance detection events out of all available distance detection events occurs or is performed by a data processing unit or module as part of the wearable or portable device, wherein the generated and stored log data correspond to one or a plurality out of the following:
-- all available distance detection events,
-- all available relevant distance detection events,
-- the continuous encounter time intervals corresponding or resulting thereof,
-- the relevant encounter events corresponding or resulting thereof.

Additionally, it is furthermore preferred that the wearable or portable device is configured to store the log data and to transmit the log data to an edge device associated to or assigned to the wearable or portable device (201, wherein the determination or selection of the relevant distance detection events out of all available distance detection events occurs or is performed by a data processing unit or module as part of the edge device, wherein the generated and stored log data correspond to one or a plurality out of the following:
-- all available distance detection events,
-- all available relevant distance detection events,
-- the continuous encounter time intervals corresponding or resulting thereof,
-- the relevant encounter events corresponding or resulting thereof.

Furthermore, the present invention relates to a system for enabling contact tracing among a plurality of persons by means of detecting relevant encounters between two persons, wherein each of the plurality of persons are wearing or carrying a wearable or portable device,
the system especially comprising, besides the plurality of wearable or portable devices, a server entity or functionality, an edge device, and computing devices,
the wearable or portable devices having or being associated or assigned to, respectively, a tag identifier, the tag identifiers being repeatedly broadcast by the wearable or portable devices, respectively, using a wireless communication interface of the wearable or portable devices,
wherein, in a contact situation of a specific wearable or portable device with a specific further wearable or portable device, the wearable or portable device is able, by means of the wearable or portable device's wireless communication interface and with respect to the further wearable or portable device, to determine repeatedly the distance, at different points in time, towards the further wearable or portable device and to receive the further wearable or portable device's tag identifier, thereby generating and storing log data of the contact situation of the wearable or portable device with the further wearable or portable device,
wherein in order to detect relevant encounters of the wearable or portable device with the further wearable or portable device the system is configured such that:
   -- an available plurality of relevant distance detection events involving the further wearable or portable device, detected by the wearable or portable device, is determined by means of - among all available distance detection events of the further wearable or portable device, detected by the wearable or portable device - considering or selecting only such distance detection events indicating the further wearable or portable device to be positioned at a distance, from the wearable or portable device, of below a predetermined distance threshold or at most equal to the predetermined distance threshold,
   -- regarding the available plurality of relevant distance detection events, involving the further wearable or portable device and detected by the wearable or portable device, one or a plurality of continuous encounter time intervals are determined or generated regarding chronologically supposedly continuous encounters - of at maximum the predetermined distance threshold between the wearable or portable device and the further wearable or portable device -, wherein each continuous encounter time interval corresponds to a certain number of relevant distance detection events, each of whose comprising or being assigned to a respective point in time of the respective distance detection event, the duration of the continuous encounter time interval being defined as or being determined to correspond to the time difference between the most recent and the earliest of these points in time, respectively,
   -- based on the one or the plurality of detected or generated continuous encounter time intervals regarding the further wearable or portable device, an encounter duration is determined, the encounter duration corresponding to the sum of the durations of the continuous encounter time intervals,
wherein a relevant encounter is detected in case that the encounter duration exceeds a predetermined duration threshold.

Furthermore, the present invention relates to an edge device and to wearable or portable devices for enabling contact tracing among a plurality of persons by means of detecting relevant encounters between two persons, wherein each of the plurality of persons are wearing or carrying a wearable or portable device,
the wearable or portable devices having or being associated or assigned to, respectively, a tag identifier, the tag identifiers being repeatedly broadcast by the wearable or portable devices, respectively, using a wireless communication interface of the wearable or portable devices,
wherein, in a contact situation of a specific wearable or portable device with a specific further wearable or portable device, the wearable or portable device is able, by means of the wearable or portable device's wireless communication interface and with respect to the further wearable or portable device, to determine repeatedly the distance, at different points in time, towards the further wearable or portable device and to receive the further wearable or portable device's tag identifier, thereby generating and storing log data of the contact situation of the wearable or portable device with the further wearable or portable device,
wherein in order to detect relevant encounters of the wearable or portable device with the further wearable or portable device the edge device and the wearable or portable devices are configured such that:
   -- an available plurality of relevant distance detection events involving the further wearable or portable device, detected by the wearable or portable device, is determined by means of - among all available distance detection events of the further wearable or portable device, detected by the wearable or portable device - considering or selecting only such distance detection events indicating the further wearable or portable device to be positioned at a distance, from the wearable or portable device, of below a predetermined distance threshold or at most equal to the predetermined distance threshold,
   -- regarding the available plurality of relevant distance detection events, involving the further wearable or portable device and detected by the wearable or portable device, one or a plurality of continuous encounter time intervals are determined or generated regarding chronologically supposedly continuous encounters - of at maximum the predetermined distance threshold between the wearable or portable device and the further wearable or portable device -, wherein each continuous encounter time interval corresponds to a certain number of relevant distance detection events, each of whose comprising or being assigned to a respective point in time of the respective distance detection event, the duration of the continuous encounter time interval being defined as or being determined to correspond to the time difference between the most recent and the earliest of these points in time, respectively,
   -- based on the one or the plurality of detected or generated continuous encounter time intervals regarding the further wearable or portable device, an encounter duration is determined, the encounter duration corresponding to the sum of the durations of the continuous encounter time intervals,
wherein a relevant encounter is detected in case that the encounter duration exceeds a predetermined duration threshold.

Additionally, the present invention relates to wearable or portable devices for enabling contact tracing among a plurality of persons by means of detecting relevant encounters between two persons, wherein each of the plurality of persons are wearing or carrying a wearable or portable device equipped with a data processing unit or module, the wearable or portable devices having or being associated or assigned to, respectively, a tag identifier, the tag identifiers being repeatedly broadcast by the wearable or portable devices, respectively, using a wireless communication interface of the wearable or portable devices,
wherein, in a contact situation of a specific wearable or portable device with a specific further wearable or portable device, the wearable or portable device is able, by means of the wearable or portable device's wireless communication interface and with respect to the further wearable or portable device, to determine repeatedly the distance, at different points in time, towards the further wearable or portable device and to receive the further wearable or portable device's tag identifier, thereby generating and storing log data of the contact situation of the wearable or portable device with the further wearable or portable device,
wherein in order to detect relevant encounters of the wearable or portable device with the further wearable or portable device the wearable or portable devices are configured such that:
   -- an available plurality of relevant distance detection events involving the further wearable or portable device, detected by the wearable or portable device, is determined, by the data processing unit or module of the wearable or portable device, by means of - among all available distance detection events of the further wearable or portable device, detected by the wearable or portable device - considering or selecting only such distance detection events indicating the further wearable or portable device to be positioned at a distance, from the wearable or portable device, of below a predetermined distance threshold or at most equal to the predetermined distance threshold,
   -- regarding the available plurality of relevant distance detection events, involving the further wearable or portable device and detected by the wearable or portable device, one or a plurality of continuous encounter time intervals are determined or generated regarding chronologically supposedly continuous encounters - of at maximum the predetermined distance threshold between the wearable or portable device and the further wearable or portable device -, wherein each continuous encounter time interval corresponds to a certain number of relevant distance detection events, each of whose comprising or being assigned to a respective point in time of the respective distance detection event, the duration of the continuous encounter time interval being defined as or being determined to correspond to the time difference between the most recent and the earliest of these points in time, respectively,
   -- based on the one or the plurality of detected or generated continuous encounter time intervals regarding the further wearable or portable device, an encounter duration is determined, the encounter duration corresponding to the sum of the durations of the continuous encounter time intervals,
wherein a relevant encounter is detected in case that the encounter duration exceeds a predetermined duration threshold.

Additionally, the present invention relates to a program comprising a computer readable program code which, when executed on a computer and/or on a computing device and/or on a server entity or functionality and/or on an edge device and/or on a wearable or portable device, or in part on a computing device and/or in part on a server entity or functionality and/or in part on an edge device and/or on a wearable or portable device, causes the computer and/or the computing device and/or the server entity or functionality and/or the edge device and/or the wearable or portable device to perform the inventive method.

Still additionally, the present invention relates to a computer-readable medium comprising instructions which when executed on a computer and/or on a computing device and/or on a server entity or functionality and/or on an edge device and/or on a wearable or portable device, or in part on a computing device and/or in part on a server entity or functionality and/or in part on an edge device and/or on a wearable or portable device, causes the computer and/or the computing device and/or the server entity or functionality and/or the edge device and/or the wearable or portable device to perform the inventive method.

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference figures quoted below refer to the attached The scope of protection is defined by the appendend claims.

### BRIEF DESCRIPTION OF THE DRAWINGS

Figure 1 schematically illustrates a plurality of persons in a contact detection and/or contact tracing setting, wherein each of the plurality of persons is wearing or carrying a wearable or portable device (or tag) of a plurality of wearable or portable devices (or tags).
Figure 2 schematically illustrates a situation of a plurality of wearable or portable devices (or tags) being connected to an edge device, and the edge device communicating with a server entity or functionality.

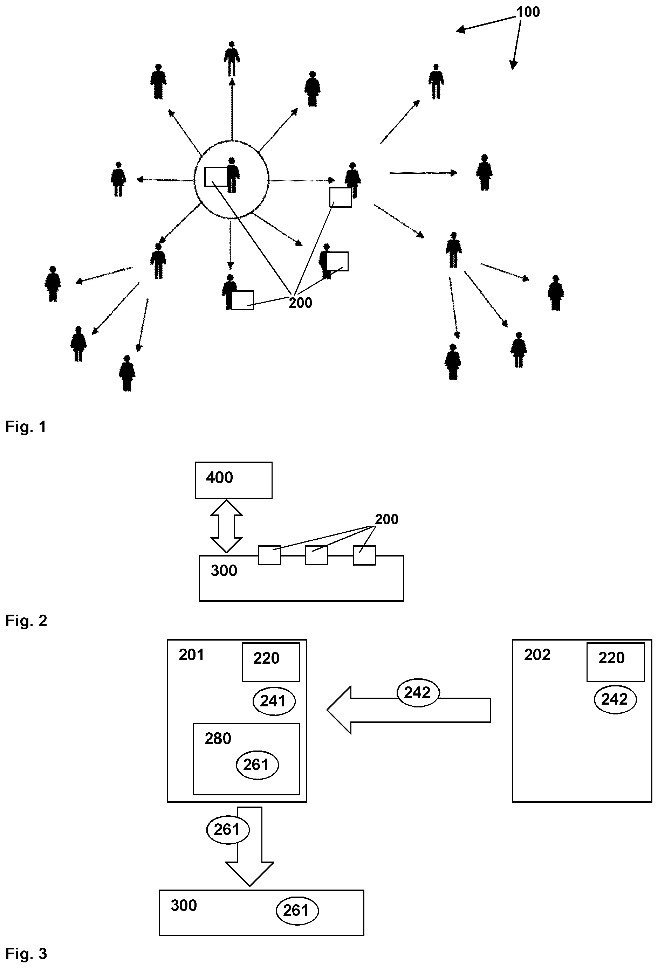

Figure 3 schematically illustrates a contact situation of a wearable or portable device with a further wearable or portable device, the wearable or portable device being able, by means of the wearable or portable device's wireless communication interface and with respect to the further wearable or portable device, to determine repeatedly the distance towards the further wearable or portable device and to receive the further wearable or portable device's tag identifier, thereby generating and storing log data of the contact situation of the wearable or portable device with the further wearable or portable device.
Figure 4 schematically illustrates an exemplary situation according to the present invention for determining or detecting a relevant encounter out of the log data.

### DETAILED DESCRIPTION

The present invention will be described with respect to particular embodiments and with reference to certain drawings, but the invention is not limited thereto but only by the claims. The drawings described are only schematic and are nonlimiting. In the drawings, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In Figure 1, a plurality of persons 100 in a contact detection and/or contact tracing setting are schematically represented, wherein each of the plurality of persons 100 is wearing or carrying a wearable or portable device (or tag) of a plurality of wearable or portable devices (or tags) 200, wherein for the sake of simplicity, Figure 1 only shows wearable or portable devices 200 for four persons 100 explicitly.

In Figure 2, a situation of a plurality of wearable or portable devices (or tags) 200 being connected to an edge device 300 is schematically shown, wherein the edge device 300 is communicating with a server entity or functionality 400.

In Figure 3, a contact situation of a (specific) wearable or portable device 201 with a (specific) further wearable or portable device 202 is schematically shown. Each of the wearable or portable devices 201, 202 comprise, respectively, a wireless communication interface 220, and each of the wearable or portable devices 201, 202 comprise, respectively, a tag identifier, the wearable or portable device's tag identifier being indicated, in Figure 3, by means of reference sign 241, and the further wearable or portable device's tag identifier being indicated by means of reference sign 242. By means of the wearable or portable device's wireless communication interface 220, the wearable or portable device 201 is able, with respect to the further wearable or portable device 202, to determine repeatedly the or its distance (i.e. the distance of the wearable or portable device 201) towards the further wearable or portable device 202 and to receive the further wearable or portable device's tag identifier 242 (by means of receiving radio frequency signals broadcast by the further wearable or portable device 202 and comprising the further wearable or portable device's tag identifier 242), thereby generating and storing log data 261 of the contact situation of the wearable or portable device 201 with the further wearable or portable device 202. Of course, analogous contact situations will typically also arise with respect to still further wearable or portable device (not individually indicated by means of references signs), i.e. the wearable or portable device 201 will typically also receive the broadcast signals of these still further wearable or portable devices, and, consequently, the log data 261 will typically also comprise information regarding the corresponding contact situations.

Figure 3 furthermore schematically shows the log data 261 of the wearable or portable device 201 being stored in a suitable memory device or module of the wearable or portable device 201 (i.e. the wearable or portable device 201 comprises such a suitable memory device or module 280, only represented in Figure 3 for the wearable or portable device 201) and being transferred to the edge device 300, shown in Figure 2. According to one embodiment of the present invention, the log data 261 (of the wearable or portable device 201) are transferred to the edge device 300, typically once the wearable or portable device 201 is - after having been used by a person, e.g. during a work shift or during a working day - put back to or with the edge device 300, especially for purposes of charging (the batteries of the wearable or portable device 201) - a situation that is schematically shown in Figure 2.

As has been explained especially with respect to Figure 3, in a contact situation of the wearable or portable device 201 with the further wearable or portable device 202, the wearable or portable device 201 repeatedly (i.e. at different points in time) determines the distance towards the further wearable or portable device 202 and receives the further wearable or portable device's tag identifier 242, thereby generating and storing the log data 261 of especially the contact situation of the wearable or portable device 201 with the further wearable or portable device 202 (typically not only thereof but likely also information regarding additional contact situations of the wearable or portable device 201 with still further wearable or portable device, i.e. this information (i.e. the respective distance data for different points in time and the still further wearable or portable device's tag identifiers) is typically also part of the log data 261). After the wearable or portable device 201 has been used (i.e. worn by the respective user or person of the plurality of persons 100), the wearable or portable device 201 is typically put back to the edge device 300 and the log data 261 transferred to the edge device 300 in a protected manner, i.e. protected especially against eavesdropping the transmission of the - especially unencrypted log data 261 to the edge device 300.

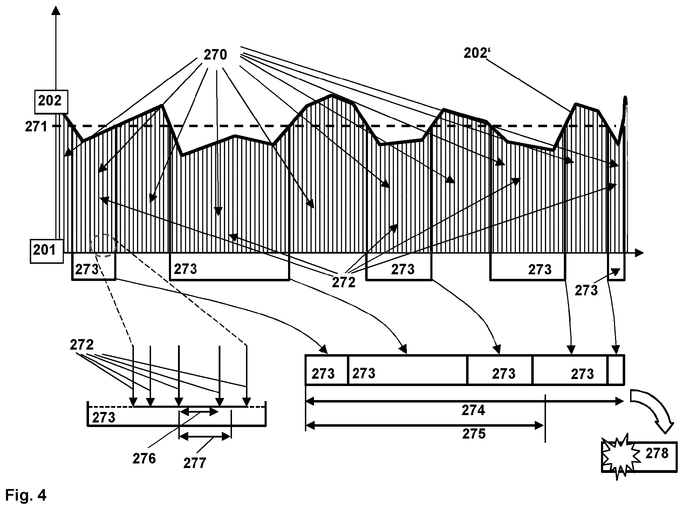

In Figure 4, an exemplary embodiment of the method according to the present invention is schematically shown by means of schematically illustrating an exemplary situation according to the present invention for determining or detecting a relevant encounter out of the log data. Figure 4 shows the situation of the wearable or portable device 201 and the further wearable or portable device 202 being positioned relative to one another such that the wearable or portable device 201 receives the broadcasts emitted by the further wearable or portable device 202. The abscissa in Figure 4 corresponds to time and the ordinate refers to the distance of the further wearable or portable device 202 from the wearable or portable device 201.

The broadcasts emitted by the further wearable or portable device 202 (comprising the further wearable or portable device's tag identifier 242) are schematically indicated by means of thin vertical lines, and the distance of the further wearable or portable device 202 is varying according to the curve 202' shown.

The thin vertical lines correspond to distance detection events 270, i.e. occasions of successful determination (by the wearable or portable device 201) of the distance towards the further wearable or portable device 202. The distance detection events 270 refer to all such occasions of successful distance determination during the considered time interval, i.e. the distance detection events 270 correspond to all log data 261 generated, by the wearable or portable device 201, with respect to the further wearable or portable device 202. Of these distance detection events 270, only a part are relevant distance detection events 272.

According to the present invention, the relevant distance detection events 272 are determined in a first step: an available plurality of relevant distance detection events 272 (involving the further wearable or portable device 202, and detected by the wearable or portable device 201) is determined by means of - among all available distance detection events 270 (of the further wearable or portable device 202, detected by the wearable or portable device 201) - considering or selecting only such distance detection events indicating the further wearable or portable device 202 to be positioned at a distance, from the wearable or portable device 201, of below a predetermined distance threshold 271 or at most equal to the predetermined distance threshold 271. The predetermined distance threshold 271 is indicated in Figure 4 by means of a dashed line 271. During time intervals of the further wearable or portable device 202 being closer to the wearable or portable device 201 (i.e. the line 202' is below the dashed line 271) the corresponding (recorded) distance detection events correspond to relevant distance detection events 272, whereas detections during other time intervals are not considered relevant distance detection events 272.

In a second step, regarding the available plurality of relevant distance detection events 272 (involving the further wearable or portable device 202 and detected by the wearable or portable device 201), one or a plurality of continuous encounter time intervals 273 are determined or generated regarding chronologically supposedly continuous encounters (of at maximum the predetermined distance threshold 271 between the wearable or portable device 201 and the further wearable or portable device 202), wherein each continuous encounter time interval 273 corresponds to a certain number of relevant distance detection events 272, each of whose comprising or being assigned to a respective point in time of the respective distance detection event 272, the duration of the continuous encounter time interval 273 being defined as or being determined to correspond to the time difference between the most recent and the earliest of these points in time, respectively. In other words, all consecutive relevant distance detection events 272 are combined to collectively form a continuous encounter time interval 273, whose duration corresponds to the time difference between the most recent and the earliest of the corresponding (relevant) distance detection events.

In a third step, based on the one or the plurality of detected or generated continuous encounter time intervals 273 (regarding the further wearable or portable device 202), an encounter duration 274 (or total encounter duration 274) is determined, the encounter duration 274 corresponding to the sum of the durations of the continuous encounter time intervals 273. A relevant encounter (typically leading to a relevant encounter event 278) is detected in case that the encounter duration 274 exceeds a predetermined duration threshold 275.

According to a preferred embodiment of the present invention, the available plurality of relevant distance detection events 272, involving the further wearable or portable device 202 and detected by the wearable or portable device 201, a chronologically continuous encounter is supposed to have occurred and, correspondingly, a continuous encounter time interval 273 is determined or generated in case that the maximum time difference 276 of consecutively detected or logged distance detection events - regarding their respective points in time in consecutive order - is smaller than or equal to a maximum gap duration 277. This is illustrated in the lower left hand part of Figure 4 for a couple of relevant distance detection events 272 which are represented in more detail. This representation shows that distance detection events might not always occur (i.e. corresponding broadcast signals being (correctly) received (and correctly processed) by the wearable or portable device 201) extremely regularly (according to the normal or adapted transmission rate) but there could be time gaps in the distance detection events such that between two successive distance detection events there might be 1 second or 2 seconds in one case and between two other successive distance detection events there might be 4 or 5 seconds. According to the present invention, while defining (or generating) the continuous encounter time interval 273, only such relevant distance detection events 272 are combined in a continuous encounter time interval 273 whose maximum time difference 276 of pairs of successive relevant distance detection events 272 is inferior to (or does not exceed) the predefined maximum gap duration 277.

According to the present invention, in the first step, the plurality of relevant distance detection events 272 is determined. This is especially done by means of considering all available distance detection events 270 related to the further wearable or portable device 202, either continuously or at the end of a logging time interval, wherein the logging time interval especially corresponds to, or approximately corresponds to, one of out of the following: one day or 24 hours, one work shift, 16 hours or 12 hours or 10 hours or 9 hours or 8 hours or 6 hours or 4 hours, 3 hours or 2 hours or 1 hour. In case that the wearable or portable devices 200 are provided as tags (i.e. typically not equipped with a powerful computing functionality such as, e.g., provided by a personal device such as a smart phone or a tablet or other computing device), such tags are typically worn by a person for a certain purpose, e.g., a work shift or the like, and typically returned to a location of an edge device 300. In such a use scenario, the time of returning such a tag device to the edge device 300 is the natural occasion to analyze and to determine the relevant distance detection events 272 from the further non-relevant distance detection events of the totality of distance detection events 270. This provides the advantage that by means of the edge device 300, the log data 261 are able to be transported safely and in a protected manner; additionally, the batteries of such tag devices do not need to be drained by transmitting the log data 261. However, in case that the wearable or portable devices 200 are provided with data processing functionalities (units or module) as part of the wearable or portable device 201, it is advantageously possible to more frequently analyze and determine the relevant distance detection events 272 (out of the totality of distance detection events 270). Thereby, it is advantageously possible to strongly reduce the required data volumes to store the log data 261.

According to further preferred embodiments of the present invention, in case that a relevant encounter is detected, a relevant encounter event 278 is generated. Such a relevant encounter event 278 especially comprises or is related to - especially besides the wearable or portable device's tag identifier 241 and the further wearable or portable device's tag identifier 242 - at least one out of the following:
-- all corresponding continuous encounter time intervals 273 related to the relevant encounter event 278,
-- the encounter duration 274, derived from all corresponding continuous encounter time intervals 273 related to the relevant encounter event 278, as well as the respective earliest as well as most recent points in time of the overall time span of the relevant encounter event 278,
-- the encounter duration 274, derived from all corresponding continuous encounter time intervals 273 related to the relevant encounter event 278, as well as the overall time span of the relevant encounter event 278 and an indicative time stamp information of either the beginning, or the end or the middle of the overall time span of the relevant encounter event 278,
-- the encounter duration 274, derived from all corresponding continuous encounter time intervals 273 related to the relevant encounter event 278.

Hence, according to the present invention, it is advantageously possible to reduce the log data 261 to such an extent that only the relevant (binary) encounter events 278 are retained. Hence, a wearable or portable device 201 could have had contacts (or distance detection events), e.g. during a day with, say, 30 other wearable or portable devices, and of these 30 other wearable or portable devices, only for (or with) 5 other wearable or portable devices, there are relevant encounter events 278 stored in the log data 261; in such a case, all remaining log data 261 of that day (to be further processed) correspond to all the relevant encounter events 278 with these 5 other wearable or portable devices.

Hence, according to the present invention, it is preferred that the initially collected (raw) log data 261 are split into sub-logs, such that each sub-log contains only contacts between two wearable or portable devices 201, 202 - where "contact" (or distance detection events 270) is preferably a quadruple <My ID, Other ID, Timestamp, Distance> - with exactly one other tag (or further wearable or portable device 202), i.e. within each sub-log, there's just a single, constant "Other ID". After that, all contacts (or distance detection events 270) with distance equal to or above the predetermined distance threshold 271 are removed to obtain the relevant distance detection events 272. The predefined distance threshold might correspond, e.g., to a value of 0,5 m or 1,0 m or 1,5 m or 1,8 m or 2,0 m or 2,5 m or 3,0 m, especially as recommended by a health authority. Then, the existence of gaps is acknowledged: While usually the time difference between consecutive contacts (or relevant distance detection events 272) is constant or rather constant (say, one second), occasionally scans may fail. This is covered by a customizable constant, which indicates the maximum tolerable gap (in seconds) such that two consecutive contacts (or relevant distance detection events 272) within a sub-log are still considered to belong to the same encounter (continuous encounter time interval 273). Hence, such an encounter (or continuous encounter time interval 273), here, is (or corresponds to) a consecutive number of contacts (or relevant distance detection events 272) that belong together in the sense that each contact before the first contact (of this considered continuous encounter time interval 273) and after the last contact (of this encounter) is more seconds away than the (predetermined constant of the) maximum gap duration. In other words: a chronologically continuous encounter is supposed to have occurred and, correspondingly, a continuous encounter time interval 273 is determined or generated in case that the maximum time difference 276 of consecutively detected or logged distance detection events - regarding their respective points in time in consecutive order - is smaller than or equal to a maximum gap duration 277). The maximum gab duration corresponds, e.g., to a time interval between 2 seconds and 30 seconds, preferably to 3 seconds, or to 4 seconds, or to 5 seconds, or to 6 seconds, or to 7 seconds, or to 8 seconds, or to 10 seconds, or to 11 seconds, or to 12 seconds, or to 13 seconds, or to 14 seconds, or to 15 seconds.

Especially, according to a preferred embodiment and in order to avoid false positives continuous encounter time intervals 273, brief encounters might be removed, such as: all encounters below a customizable predetermined short duration threshold are removed or discarded. The short duration threshold might correspond, e.g., to a time interval between 10 seconds and 5 minutes, preferably to 10 seconds, or to 15 seconds, or to 20 seconds, or to 30 seconds, or to 40 seconds, or to 50 seconds, or to 60 seconds, or to 70 seconds, or to 80 seconds, or to 90 seconds, or to 100 seconds, or to 110 seconds, or to 120 seconds.

If, by way of other channels, it is known to a user (of a wearable or portable device) which user (or person) (i.e. the respective tag IDs) at which times belong to someone who got a positive diagnosis (e.g. of Covid-19). Hence, the duration of all encounters (between two devices) can be summed up, and if the sum is above, or exceeds, the predetermined duration threshold 275, such an relevant encounter is considered as risky, and an alert is raised, triggering, e.g., the suggestion to conform to self-quarantine rules and/or performing a medical test, such as a Covid-19 test. The duration threshold 275 might correspond, e.g., to a time interval between 5 minutes and 30 minutes, preferably to 10 minutes, or to 12 minutes, or to 14 minutes, or to 15 minutes, or to 16 minutes, or to 18 minutes, or to 20 minutes, or to 25 minutes.

The approach according to the present invention counters both the problem of brief encounters (below the short duration threshold, which are ignored) and the problem that average distances over long encounters (think several hours) may be large, but a few intimate minutes are still risky.

## Claims

1. Method for enabling contact tracing among a plurality of persons (100) by means of detecting relevant encounters between two persons, wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202), wherein, in a contact situation of a specific wearable or portable device (201) with a specific further wearable or portable device (202), the wearable or portable device (201) is able, by means of the wearable or portable device's wireless communication interface (220) and with respect to the further wearable or portable device (202), to determine repeatedly the distance, at different points in time, towards the further wearable or portable device (202) and to receive the further wearable or portable device's tag identifier (242), thereby generating and storing log data (261) of the contact situation of the wearable or portable device (201) with the further wearable or portable device (202),
wherein in order to detect relevant encounters of the wearable or portable device (201) with the further wearable or portable device (202) the method comprises the following steps:
-- in a first step, an available plurality of relevant distance detection events (272) involving the further wearable or portable device (202), detected by the wearable or portable device (201), is determined by means of - among all available distance detection events (270) of the further wearable or portable device (202), detected by the wearable or portable device (201) - considering or selecting only such distance detection events indicating the further wearable or portable device (202) to be positioned at a distance, from the wearable or portable device (201), of below a predetermined distance threshold (271) or at most equal to the predetermined distance threshold (271),
-- in a second step, regarding the available plurality of relevant distance detection events (272), involving the further wearable or portable device (202) and detected by the wearable or portable device (201), one or a plurality of continuous encounter time intervals (273) are determined or generated regarding chronologically supposedly continuous encounters - of at maximum the predetermined distance threshold (271) between the wearable or portable device (201) and the further wearable or portable device (202) -, wherein each continuous encounter time interval (273) corresponds to a certain number of relevant distance detection events (272), each of whose comprising or being assigned to a respective point in time of the respective distance detection event (272), the duration of the continuous encounter time interval being defined as or being determined to correspond to the time difference between the most recent and the earliest of these points in time, respectively,
-- in a third step, based on the one or the plurality of detected or generated continuous encounter time intervals (273) regarding the further wearable or portable device (202), an encounter duration (274) is determined, the encounter duration (274) corresponding to the sum of the durations of the continuous encounter time intervals (273),
wherein a relevant encounter is detected in case that the encounter duration exceeds a predetermined duration threshold (275).

2. Method according to claim 1, wherein, regarding, during the second step, the available plurality of relevant distance detection events (272), involving the further wearable or portable device (202) and detected by the wearable or portable device (201), a chronologically continuous encounter is supposed to have occurred and, correspondingly, a continuous encounter time interval (273) is determined or generated in case that the maximum time difference (276) of consecutively detected or logged distance detection events - regarding their respective points in time in consecutive order - is smaller than or equal to a maximum gap duration (277).

3. Method according to one of the preceding claims, wherein the plurality of relevant distance detection events (272) is determined, especially by means of considering all available distance detection events (270) related to the further wearable or portable device (202), either continuously or at the end of a logging time interval, wherein the logging time interval especially corresponds to, or approximately corresponds to, one of out of the following:
-- one day or 24 hours,
-- one work shift,
-- 16 hours or 12 hours or 10 hours or 9 hours or 8 hours or 6 hours or 4 hours,
-- 3 hours or 2 hours or 1 hour.

4. Method according to one of the preceding claims, wherein in case that a relevant encounter is detected, a relevant encounter event (278) is generated, wherein especially the relevant encounter event (278) comprises or is related to - especially besides the wearable or portable device's tag identifier (241) and the further wearable or portable device's tag identifier (242) - at least one out of the following:
-- all corresponding continuous encounter time intervals (273) related to the relevant encounter event (278),
-- the encounter duration (274), derived from all corresponding continuous encounter time intervals (273) related to the relevant encounter event (278), as well as the respective earliest as well as most recent points in time of the overall time span of the relevant encounter event (278),
-- the encounter duration (274), derived from all corresponding continuous encounter time intervals (273) related to the relevant encounter event (278), as well as the overall time span of the relevant encounter event (278) and an indicative time stamp information of either the beginning, or the end or the middle of the overall time span of the relevant encounter event (278),
-- the encounter duration (274), derived from all corresponding continuous encounter time intervals (273) related to the relevant encounter event (278).

5. Method according to one of the preceding claims, wherein, as part of the log data (261) generated and stored by the wearable or portable device (201), each one of the distance detection events (270), and likewise each one of the relevant distance detection events (272), detected or generated with regard to the further wearable or portable device (202), comprises a quadruple of:
-- the wearable or portable device's tag identifier (241),
-- the further wearable or portable device's tag identifier (242),
-- a time stamp information indicative of the point in time of the respective distance detection event,
-- a distance information indicative of the detected or measured distance between the wearable or portable device (201) and the further wearable or portable device (202),
wherein especially the relevant distance detection events (272) are the ones among all available distance detection events (270) of or detected by the wearable or portable device (201) having a distance information below the predetermined distance threshold (271).

6. Method according to one of the preceding claims, wherein each one of the continuous encounter time intervals (273), generated from or based on the available distance detection events (270) and the available relevant distance detection events (272) and detected or generated with regard to both the wearable or portable device (201) and the further wearable or portable device (202), comprises both the wearable or portable device's tag identifier (241) and the further wearable or portable device's tag identifier (242), as well as:
-- either a time stamp information indicative of the beginning or and end of the respective continuous encounter time interval (273) as well as a duration information of the continuous encounter time interval (273),
-- or a time stamp information of the beginning of the respective continuous encounter time interval (273) and a time stamp information of the end of the respective continuous encounter time interval (273).

7. Method according to one of the preceding claims, wherein the determination or selection of the relevant distance detection events (272) out of all available distance detection events (270) occurs or is performed by a data processing unit or module as part of the wearable or portable device (201), wherein the generated and stored log data (261) correspond to one or a plurality out of the following:
-- all available distance detection events (270),
-- all available relevant distance detection events (272),
-- the continuous encounter time intervals (273) corresponding or resulting thereof,
-- the relevant encounter events (278) corresponding or resulting thereof.

8. Method according to one of the preceding claims, wherein the wearable or portable device (201) is configured to store the log data (261) and to transmit the log data (261) to an edge device (300) associated to or assigned to the wearable or portable device (201, wherein the determination or selection of the relevant distance detection events (272) out of all available distance detection events (270) occurs or is performed by a data processing unit or module as part of the edge device (300), wherein the generated and stored log data (261) correspond to one or a plurality out of the following:
-- all available distance detection events (270),
-- all available relevant distance detection events (272),
-- the continuous encounter time intervals (273) corresponding or resulting thereof,
-- the relevant encounter events (278) corresponding or resulting thereof.

9. System for enabling contact tracing among a plurality of persons (100) by means of detecting relevant encounters between two persons, wherein each of the plurality of persons (100) are wearing or carrying a wearable or portable device (201, 202), the system especially comprising, besides the plurality of wearable or portable devices (200), a server entity (400), an edge device (300), and computing devices, the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202),
wherein, in a contact situation of a specific wearable or portable device (201) with a specific further wearable or portable device (202), the wearable or portable device (201) is able, by means of the wearable or portable device's wireless communication interface (220) and with respect to the further wearable or portable device (202), to determine repeatedly the distance, at different points in time, towards the further wearable or portable device (202) and to receive the further wearable or portable device's tag identifier (242), thereby generating and storing log data (261) of the contact situation of the wearable or portable device (201) with the further wearable or portable device (202),
wherein in order to detect relevant encounters of the wearable or portable device (201) with the further wearable or portable device (202) the system is configured such that:
-- an available plurality of relevant distance detection events (272) involving the further wearable or portable device (202), detected by the wearable or portable device (201), is determined by means of - among all available distance detection events (270) of the further wearable or portable device (202), detected by the wearable or portable device (201) - considering or selecting only such distance detection events indicating the further wearable or portable device (202) to be positioned at a distance, from the wearable or portable device (201), of below a predetermined distance threshold (271) or at most equal to the predetermined distance threshold (271),
-- regarding the available plurality of relevant distance detection events (272), involving the further wearable or portable device (202) and detected by the wearable or portable device (201), one or a plurality of continuous encounter time intervals (273) are determined or generated regarding chronologically supposedly continuous encounters - of at maximum the predetermined distance threshold between the wearable or portable device (201) and the further wearable or portable device (202) - , wherein each continuous encounter time interval (273) corresponds to a certain number of relevant distance detection events (272), each of whose comprising or being assigned to a respective point in time of the respective distance detection event (272), the duration of the continuous encounter time interval being defined as or being determined to correspond to the time difference between the most recent and the earliest of these points in time, respectively,
-- based on the one or the plurality of detected or generated continuous encounter time intervals (273) regarding the further wearable or portable device (202), an encounter duration (274) is determined, the encounter duration (274) corresponding to the sum of the durations of the continuous encounter time intervals (273),
wherein a relevant encounter is detected in case that the encounter duration exceeds a predetermined duration threshold (275).

10. Wearable or portable devices (201, 202) for enabling contact tracing among a plurality of persons (100) by means of detecting relevant encounters between two persons, wherein each of the plurality of persons (100) are wearing or carrying a wearable or portable device (201, 202) equipped with a data processing unit or module, the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202),
wherein, in a contact situation of a specific wearable or portable device (201) with a specific further wearable or portable device (202), the wearable or portable device (201) is able, by means of the wearable or portable device's wireless communication interface (220) and with respect to the further wearable or portable device (202), to determine repeatedly the distance, at different points in time, towards the further wearable or portable device (202) and to receive the further wearable or portable device's tag identifier (242), thereby generating and storing log data (261) of the contact situation of the wearable or portable device (201) with the further wearable or portable device (202),
wherein in order to detect relevant encounters of the wearable or portable device (201) with the further wearable or portable device (202) the wearable or portable devices (201, 202) are configured such that:
-- an available plurality of relevant distance detection events (272) involving the further wearable or portable device (202), detected by the wearable or portable device (201), is determined, by the data processing unit or module of the wearable or portable device (201), by means of - among all available distance detection events (270) of the further wearable or portable device (202), detected by the wearable or portable device (201) - considering or selecting only such distance detection events indicating the further wearable or portable device (202) to be positioned at a distance, from the wearable or portable device (201), of below a predetermined distance threshold (271) or at most equal to the predetermined distance threshold (271),
-- regarding the available plurality of relevant distance detection events (272), involving the further wearable or portable device (202) and detected by the wearable or portable device (201), one or a plurality of continuous encounter time intervals (273) are determined or generated regarding chronologically supposedly continuous encounters - of at maximum the predetermined distance threshold between the wearable or portable device (201) and the further wearable or portable device (202) - , wherein each continuous encounter time interval (273) corresponds to a certain number of relevant distance detection events (272), each of whose comprising or being assigned to a respective point in time of the respective distance detection event (272), the duration of the continuous encounter time interval being defined as or being determined to correspond to the time difference between the most recent and the earliest of these points in time, respectively,
-- based on the one or the plurality of detected or generated continuous encounter time intervals (273) regarding the further wearable or portable device (202), an encounter duration (274) is determined, the encounter duration (274) corresponding to the sum of the durations of the continuous encounter time intervals (273),
wherein a relevant encounter is detected in case that the encounter duration exceeds a predetermined duration threshold (275).

11. Program comprising a computer readable program code, which, when executed on a computer and/or on a computing device (151) and/or on a server entity (400) and/or on an edge device (300) and/or on a wearable or portable device (201), or in part on a computing device (151) and/or in part on a server entity (400) and/or in part on an edge device (300) and/or on a wearable or portable device (201), causes the computer and/or the computing device (151) and/or the server entity (400) and/or the edge device (300) and/or the wearable or portable device (201) to perform a method according one of claims 1 to 8.

12. Computer-readable medium comprising instructions which when executed on a computer and/or on a computing device (151) and/or on a server entity (400) and/or on an edge device (300) and/or on a wearable or portable device (201), or in part on a computing device (151) and/or in part on a server entity (400) and/or in part on an edge device (300) and/or on a wearable or portable device (201), causes the computer and/or the computing device (151) and/or the server entity (400) and/or the edge device (300) and/or the wearable or portable device (201) to perform a method according one of claims 1 to 8.

## Patentansprüche

1. Verfahren zum Ermöglichen einer Kontaktverfolgung zwischen mehreren Personen (100) durch Detektieren relevanter Begegnungen zwischen zwei Personen, wobei jede der mehreren Personen (100) eine am Körper tragbare oder portable Vorrichtung (201, 202) am Körper trägt oder mit sich führt, wobei die am Körper tragbaren oder portablen Vorrichtungen (201, 202) jeweils eine Tag-Kennung (241, 242) aufweisen oder einer solchen zugeordnet oder zugewiesen sind, wobei die Tag-Kennungen (241, 242) jeweils wiederholt durch die am Körper tragbaren oder portablen Vorrichtungen (201, 202) unter Verwendung einer Drahtloskommunikationsschnittstelle der am Körper tragbaren oder portablen Vorrichtungen (201, 202) rundgesendet werden, wobei, in einer Kontaktsituation einer spezifischen am Körper tragbaren oder portablen Vorrichtung (201) mit einer spezifischen weiteren am Körper tragbaren oder portablen Vorrichtung (202), die am Körper tragbare oder portable Vorrichtung (201) in der Lage ist, mittels der Drahtloskommunikationsschnittstelle (220) der am Körper tragbaren oder portablen Vorrichtung und in Bezug auf die weitere am Körper tragbare oder portable Vorrichtung (202) wiederholt, zu verschiedenen Zeitpunkten, die Entfernung zu der weiteren am Körper tragbaren oder portablen Vorrichtung (202) zu bestimmen und die Tag-Kennung (242) der weiteren am Körper tragbaren oder portablen Vorrichtung zu empfangen, wodurch Protokollierungsdaten (261) der Kontaktsituation der am Körper tragbaren oder portablen Vorrichtung (201) mit der weiteren am Körper tragbaren oder portablen Vorrichtung (202) generiert und gespeichert werden,
wobei das Verfahren, um relevante Begegnungen der am Körper tragbaren oder portablen Vorrichtung (201) mit der weiteren am Körper tragbaren oder portablen Vorrichtung (202) zu detektieren, die folgenden Schritte umfasst:
- in einem ersten Schritt wird eine verfügbare Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, bestimmt, indem - unter allen verfügbaren Entfernungsdetektionsereignissen (270) der weiteren am Körper tragbaren oder portablen Vorrichtung (202), die durch die am Körper tragbare oder portable Vorrichtung (201) detektiert werden - nur solche Entfernungsdetektionsereignisse berücksichtigt oder ausgewählt werden, die angeben, dass die weitere am Körper tragbare oder portable Vorrichtung (202) in einer Entfernung von der am Körper tragbaren oder portablen Vorrichtung (201) positioniert ist, die unterhalb einer zuvor festgelegten Entfernungsschwelle (271) liegt oder höchstens gleich der zuvor festgelegten Entfernungsschwelle (271) ist,
- in einem zweiten Schritt werden, bezüglich der verfügbaren Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, ein oder mehrere kontinuierliche Begegnungszeitintervalle (273) bezüglich chronologisch mutmaßlich kontinuierlicher Begegnungen - von maximal der zuvor festgelegten Entfernungsschwelle (271) zwischen der am Körper tragbaren oder portablen Vorrichtung (201) und der weiteren am Körper tragbaren oder portablen Vorrichtung (202) bestimmt oder generiert - wobei jedes kontinuierliche Begegnungszeitintervall (273) einer gewissen Anzahl relevanter Entfernungsdetektionsereignisse (272) entspricht, von denen jedes einen jeweiligen Zeitpunkt des jeweiligen Entfernungsdetektionsereignisses (272) umfasst oder einem solchen Zeitpunkt zugewiesen ist, wobei die Dauer des kontinuierlichen Begegnungszeitintervalls jeweils als die Zeitdifferenz zwischen dem neuesten und dem frühesten dieser Zeitpunkte definiert ist oder als dieser Zeitdifferenz entsprechend bestimmt wird,
- in einem dritten Schritt wird auf der Grundlage des einen oder der mehreren detektierten oder generierten kontinuierlichen Begegnungszeitintervalle (273) bezüglich der weiteren am Körper tragbaren oder portablen Vorrichtung (202) eine Begegnungsdauer (274) bestimmt, wobei die Begegnungsdauer (274) der Summe der Dauern der kontinuierlichen Begegnungszeitintervalle (273) entspricht,
wobei eine relevante Begegnung für den Fall detektiert wird, dass die Begegnungsdauer eine zuvor festgelegte Dauerschwelle (275) überschreitet.

2. Verfahren nach Anspruch 1, wobei bezüglich, während des zweiten Schrittes, der verfügbaren Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, angenommen wird, dass eine chronologisch kontinuierliche Begegnung stattgefunden hat, und dementsprechend ein kontinuierliches Begegnungszeitintervall (273) für den Fall bestimmt oder generiert wird, dass die maximale Zeitdifferenz (276) aufeinanderfolgender detektierter oder protokollierter Entfernungsdetektionsereignisse - bezüglich ihrer jeweiligen Zeitpunkte in aufeinanderfolgender Reihenfolge - kleiner als eine maximale oder gleich einer maximalen Abstandsdauer (277) ist.

3. Verfahren nach einem der vorangehenden Ansprüche, wobei die Mehrzahl relevanter Entfernungsdetektionsereignisse (272) insbesondere unter Berücksichtigung aller verfügbaren Entfernungsdetektionsereignisse (270) in Bezug auf die weitere tragbare oder portable Vorrichtung (202) entweder kontinuierlich oder am Ende eines Protokollierungszeitintervalls bestimmt wird, wobei das Protokollierungszeitintervall insbesondere einem von Folgendem entspricht oder annähernd entspricht:
- einem Tag oder 24 Stunden,
- einer Arbeitsschicht,
- 16 Stunden oder 12 Stunden oder 10 Stunden oder 9 Stunden oder 8 Stunden oder 6 Stunden oder 4 Stunden,
- 3 Stunden oder 2 Stunden oder 1 Stunde.

4. Verfahren nach einem der vorangehenden Ansprüche, wobei in dem Fall, dass eine relevante Begegnung detektiert wird, ein relevantes Begegnungsereignis (278) generiert wird, wobei insbesondere das relevante Begegnungsereignis (278) - insbesondere neben der Tag-Kennung (241) der am Körper tragbaren oder portablen Vorrichtung und der Tag-Kennung (242) der weiteren am Körper tragbaren oder portablen Vorrichtung - mindestens eines von Folgendem umfasst oder darauf bezieht:
- alle entsprechenden kontinuierlichen Begegnungszeitintervalle (273), die sich auf das relevante Begegnungsereignis (278) beziehen,
- die Begegnungsdauer (274), die aus allen entsprechenden kontinuierlichen Begegnungszeitintervallen (273) abgeleitet ist, die sich auf das relevante Begegnungsereignis (278) beziehen, sowie die jeweiligen frühesten und jüngsten Zeitpunkte der Gesamtzeitspanne des relevanten Begegnungsereignisses (278),
- die Begegnungsdauer (274), die aus allen entsprechenden kontinuierlichen Begegnungszeitintervallen (273) abgeleitet ist, die sich auf das relevante Begegnungsereignis (278) beziehen, sowie die Gesamtzeitspanne des relevanten Begegnungsereignisses (278) und eine indikative Zeitstempelinformation entweder des Beginns oder des Endes oder der Mitte der Gesamtzeitspanne des relevanten Begegnungsereignisses (278),
- die Begegnungsdauer (274), die aus allen entsprechenden kontinuierlichen Begegnungszeitintervallen (273) abgeleitet ist, die sich auf das relevante Begegnungsereignis (278) beziehen.

5. Verfahren nach einem der vorangehenden Ansprüche, wobei, als Teil der durch die am Körper tragbare oder portable Vorrichtung (201) generierten und gespeicherten Protokollierungsdaten (261), jedes der Entfernungsdetektionsereignisse (270) und gleichermaßen jedes der relevanten Entfernungsdetektionsereignisse (272), die in Bezug auf die weitere am Körper tragbare oder portable Vorrichtung (202) detektiert oder generiert werden, ein Vierfaches von Folgendem umfasst:
- der Tag-Kennung (241) der am Körper tragbaren oder portablen Vorrichtung,
- der Tag-Kennung (242) der weiteren am Körper tragbaren oder portablen Vorrichtung,
- einer Zeitstempelinformation, die den Zeitpunkt des jeweiligen Entfernungsdetektionsereignisses angibt,
- einer Entfernungsinformation, die die detektierte oder gemessene Entfernung zwischen der am Körper tragbaren oder portablen Vorrichtung (201) und der weiteren am Körper tragbaren oder portablen Vorrichtung (202) angibt,
wobei insbesondere die relevanten Entfernungsdetektionsereignisse (272) diejenigen unter allen verfügbaren Entfernungsdetektionsereignissen (270) der am Körper tragbaren oder portablen Vorrichtung (201), oder die durch die am Körper tragbare oder portable Vorrichtung (201) detektiert wurden, sind, die eine Entfernungsinformation unterhalb der zuvor festgelegten Entfernungsschwelle (271) aufweisen.

6. Verfahren nach einem der vorangehenden Ansprüche, wobei jedes der kontinuierlichen Begegnungszeitintervalle (273), die aus oder basierend auf den verfügbaren Entfernungsdetektionsereignissen (270) und den verfügbaren relevanten Entfernungsdetektionsereignissen (272) generiert werden und in Bezug sowohl auf die am Körper tragbare oder portable Vorrichtung (201) als auch die weitere am Körper tragbare oder portable Vorrichtung (202) detektiert oder generiert werden, sowohl die Tag-Kennung (241) der am Körper tragbaren oder portablen Vorrichtung als auch die Tag-Kennung (242) der weiteren am Körper tragbaren oder portablen Vorrichtung umfasst, sowie:
- entweder eine Zeitstempelinformation, die den Beginn oder das Ende des jeweiligen kontinuierlichen Begegnungszeitintervalls (273) angibt, sowie eine Dauerinformation des kontinuierlichen Begegnungszeitintervalls (273),
- oder eine Zeitstempelinformation über den Beginn des jeweiligen kontinuierlichen Begegnungszeitintervalls (273) und eine Zeitstempelinformation über das Ende des jeweiligen kontinuierlichen Begegnungszeitintervalls (273).

7. Verfahren nach einem der vorangehenden Ansprüche, wobei die Bestimmung oder Auswahl der relevanten Entfernungsdetektionsereignisse (272) aus allen verfügbaren Entfernungsdetektionsereignissen (270) durch eine Datenverarbeitungseinheit oder ein Datenverarbeitungsmodul als Teil der am Körper tragbaren oder portablen Vorrichtung (201) erfolgt oder durchgeführt wird, wobei die generierten und gespeicherten Protokollierungsdaten (261) einem oder mehreren von Folgendem entsprechen:
- allen verfügbaren Entfernungsdetektionsereignissen (270),
- allen verfügbaren relevanten Entfernungsdetektionsereignissen (272),
- den kontinuierlichen Begegnungszeitintervallen (273), die diesen entsprechen oder daraus resultieren,
- den relevanten Begegnungsereignissen (278), die diesen entsprechen oder daraus resultieren.

8. Verfahren nach einem der vorangehenden Ansprüche, wobei die am Körper tragbare oder portable Vorrichtung (201) dazu eingerichtet ist, die Protokollierungsdaten (261) zu speichern und die Protokollierungsdaten (261) an eine Edge-Vorrichtung (300) zu senden, die der am Körper tragbaren oder portablen Vorrichtung (201) zugeordnet oder zugewiesen ist, wobei die Bestimmung oder Auswahl der relevanten Entfernungsdetektionsereignisse (272) aus allen verfügbaren Entfernungsdetektionsereignissen (270) durch eine Datenverarbeitungseinheit oder ein Datenverarbeitungsmodul als Teil der Edge-Vorrichtung (300) erfolgt oder durchgeführt wird, wobei die generierten und gespeicherten Protokollierungsdaten (261) einem oder mehreren von Folgendem entsprechen:
- allen verfügbaren Entfernungsdetektionsereignissen (270),
- allen verfügbaren relevanten Entfernungsdetektionsereignissen (272),
- den kontinuierlichen Begegnungszeitintervallen (273), die diesen entsprechen oder daraus resultieren,
- den relevanten Begegnungsereignissen (278), die diesen entsprechen oder daraus resultieren.

9. System zum Ermöglichen einer Kontaktverfolgung zwischen mehreren Personen (100) mittels Detektieren relevanter Begegnungen zwischen zwei Personen, wobei jede der mehreren Personen (100) eine am Körper tragbare oder portable Vorrichtung (201, 202) am Körper trägt oder mit sich führt, wobei das System insbesondere neben den mehreren am Körper tragbaren oder portablen Vorrichtungen (200) eine Server-Entität (400), eine Edge-Vorrichtung (300) und Rechenvorrichtungen umfasst, wobei die am Körper tragbaren oder portablen Vorrichtungen (201, 202) jeweils eine Tag-Kennung (241, 242) aufweisen oder einer solchen zugeordnet oder zugewiesen sind, wobei die Tag-Kennungen (241, 242) jeweils wiederholt durch die am Körper tragbaren oder portablen Vorrichtungen (201, 202) unter Verwendung einer Drahtloskommunikationsschnittstelle der am Körper tragbaren oder portablen Vorrichtungen (201, 202) rundgesendet werden,
wobei in einer Kontaktsituation einer spezifischen am Körper tragbaren oder portablen Vorrichtung (201) mit einer spezifischen weiteren am Körper tragbaren oder portablen Vorrichtung (202) die am Körper tragbare oder portable Vorrichtung (201) in der Lage ist, mittels der Drahtloskommunikationsschnittstelle (220) der am Körper tragbaren oder portablen Vorrichtung und in Bezug auf die weitere am Körper tragbare oder portable Vorrichtung (202) wiederholt die Distanz, an verschiedenen Zeitpunkten, zu der weiteren am Körper tragbaren oder portablen Vorrichtung (202) zu bestimmen und die Tag-Kennung (242) der weiteren am Körper tragbaren oder portablen Vorrichtung zu empfangen, wodurch Protokollierungsdaten (261) der Kontaktsituation der am Körper tragbaren oder portablen Vorrichtung (201) mit der weiteren am Körper tragbaren oder portablen Vorrichtung (202) generiert und gespeichert werden,
wobei, um relevante Begegnungen der am Körper tragbaren oder portablen Vorrichtung (201) mit der weiteren am Körper tragbaren oder portablen Vorrichtung (202) zu detektieren, das System so eingerichtet ist, dass:
- eine verfügbare Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, bestimmt wird, indem - unter allen verfügbaren Entfernungsdetektionsereignissen (270) der weiteren am Körper tragbaren oder portablen Vorrichtung (202), die durch die am Körper tragbare oder portable Vorrichtung (201) detektiert werden - nur solche Entfernungsdetektionsereignisse berücksichtigt oder ausgewählt werden, die angeben, dass die weitere am Körper tragbare oder portable Vorrichtung (202) in einer Entfernung von der am Körper tragbaren oder portablen Vorrichtung (201) positioniert ist, die unterhalb einer zuvor festgelegten Entfernungsschwelle (271) liegt oder höchstens gleich der zuvor festgelegten Entfernungsschwelle (271) ist,
- bezüglich der verfügbaren Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, ein oder mehrere kontinuierliche Begegnungszeitintervalle (273) bezüglich chronologisch mutmaßlich kontinuierlicher Begegnungen - von maximal der zuvor festgelegten Entfernungsschwelle (271) zwischen der am Körper tragbaren oder portablen Vorrichtung (201) und der weiteren am Körper tragbaren oder portablen Vorrichtung (202) - bestimmt oder generiert werden, wobei jedes kontinuierliche Begegnungszeitintervall (273) einer gewissen Anzahl relevanter Entfernungsdetektionsereignisse (272) entspricht, von denen jedes einen jeweiligen Zeitpunkt des jeweiligen Entfernungsdetektionsereignisses (272) umfasst oder einem solchen Zeitpunkt zugewiesen ist, wobei die Dauer des kontinuierlichen Begegnungszeitintervalls jeweils als die Zeitdifferenz zwischen dem jüngsten und dem frühesten dieser Zeitpunkte definiert ist oder als dieser Zeitdifferenz entsprechend bestimmt wird,
- auf der Grundlage des einen oder der mehreren detektierten oder generierten kontinuierlichen Begegnungszeitintervalle (273) bezüglich der weiteren am Körper tragbaren oder portablen Vorrichtung (202) eine Begegnungsdauer (274) bestimmt wird, wobei die Begegnungsdauer (274) der Summe der Dauern der kontinuierlichen Begegnungszeitintervalle (273) entspricht, wobei eine relevante Begegnung detektiert wird, falls die Begegnungsdauer eine zuvor festgelegte Dauerschwelle (275) überschreitet.

10. Am Körper tragbare oder portable Vorrichtungen (201, 202) zum Ermöglichen einer Kontaktverfolgung zwischen mehreren Personen (100) durch Detektieren relevanter Begegnungen zwischen zwei Personen, wobei jede der mehreren Personen (100) eine am Körper tragbare oder portable Vorrichtung (201, 202) am Körper trägt oder mit sich führt, die mit einer Datenverarbeitungseinheit oder einem Datenverarbeitungsmodul ausgestattet ist, wobei die am Körper tragbaren oder portablen Vorrichtungen (201, 202) jeweils eine Tag-Kennung (241, 242) aufweisen oder einer solchen zugeordnet oder zugewiesen sind, wobei die Tag-Kennungen (241, 242) jeweils wiederholt durch die am Körper tragbaren oder portablen Vorrichtungen (201, 202) unter Verwendung einer Drahtloskommunikationsschnittstelle der am Körper tragbaren oder portablen Vorrichtungen (201, 202) rundgesendet werden,
wobei in einer Kontaktsituation einer spezifischen am Körper tragbaren oder portablen Vorrichtung (201) mit einer spezifischen weiteren am Körper tragbaren oder portablen Vorrichtung (202) die am Körper tragbare oder portable Vorrichtung (201) in der Lage ist, mittels der Drahtloskommunikationsschnittstelle (220) der am Körper tragbaren oder portablen Vorrichtung und in Bezug auf die weitere am Körper tragbare oder portable Vorrichtung (202) wiederholt die Distanz, an verschiedenen Zeitpunkten, zu der weiteren am Körper tragbaren oder portablen Vorrichtung (202) zu bestimmen und die Tag-Kennung (242) der weiteren am Körper tragbaren oder portablen Vorrichtung zu empfangen, wodurch Protokollierungsdaten (261) der Kontaktsituation der am Körper tragbaren oder portablen Vorrichtung (201) mit der weiteren am Körper tragbaren oder portablen Vorrichtung (202) generiert und gespeichert werden,
wobei, um relevante Begegnungen der am Körper tragbaren oder portablen Vorrichtung (201) mit der weiteren am Körper tragbaren oder portablen Vorrichtung (202) zu detektieren, die am Körper tragbaren oder portablen Vorrichtungen (201, 202) so eingerichtet sind, dass:
- eine verfügbare Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, durch die Datenverarbeitungseinheit oder das Datenverarbeitungsmodul der am Körper tragbaren oder portablen Vorrichtung (201) bestimmt wird, indem - unter allen verfügbaren Entfernungsdetektionsereignissen (270) der weiteren am Körper tragbaren oder portablen Vorrichtung (202), die durch die am Körper tragbare oder portable Vorrichtung (201) detektiert werden - nur solche Entfernungsdetektionsereignisse berücksichtigt oder ausgewählt werden, die angeben, dass die weitere am Körper tragbare oder portable Vorrichtung (202) in einer Entfernung von der am Körper tragbaren oder portablen Vorrichtung (201) positioniert ist, die unterhalb einer zuvor festgelegten Entfernungsschwelle (271) liegt oder höchstens gleich der zuvor festgelegten Entfernungsschwelle (271) ist,
- bezüglich der verfügbaren Mehrzahl relevanter Entfernungsdetektionsereignisse (272), an denen die weitere am Körper tragbare oder portable Vorrichtung (202) beteiligt ist und die durch die am Körper tragbare oder portable Vorrichtung (201) detektiert werden, ein oder mehrere kontinuierliche Begegnungszeitintervalle (273) bezüglich chronologisch mutmaßlich kontinuierlicher Begegnungen - von maximal der zuvor festgelegten Entfernungsschwelle (271) zwischen der am Körper tragbaren oder portablen Vorrichtung (201) und der weiteren am Körper tragbaren oder portablen Vorrichtung (202) - bestimmt oder generiert werden, wobei jedes kontinuierliche Begegnungszeitintervall (273) einer gewissen Anzahl relevanter Entfernungsdetektionsereignisse (272) entspricht, von denen jedes einen jeweiligen Zeitpunkt des jeweiligen Entfernungsdetektionsereignisses (272) umfasst oder einem solchen Zeitpunkt zugewiesen ist, wobei die Dauer des kontinuierlichen Begegnungszeitintervalls jeweils als die Zeitdifferenz zwischen dem jüngsten und dem frühesten dieser Zeitpunkte definiert ist oder als dieser Zeitdifferenz entsprechend bestimmt wird,
- auf der Grundlage des einen oder der mehreren detektierten oder generierten kontinuierlichen Begegnungszeitintervalle (273) bezüglich der weiteren am Körper tragbaren oder portablen Vorrichtung (202) eine Begegnungsdauer (274) bestimmt wird, wobei die Begegnungsdauer (274) der Summe der Dauern der kontinuierlichen Begegnungszeitintervalle (273) entspricht, wobei eine relevante Begegnung detektiert wird, falls die Begegnungsdauer eine zuvor festgelegte Dauerschwelle (275) überschreitet.

11. Programm, umfassend einen computerlesbaren Programmcode, der, wenn er auf einem Computer und/oder in einer Rechenvorrichtung (151) und/oder in einer Server-Entität (400) und/oder in einer Edge-Vorrichtung (300) und/oder in einer am Körper tragbaren oder portablen Vorrichtung (201) oder teilweise in einer Rechenvorrichtung (151) und/oder teilweise in einer Server-Entität (400) und/oder teilweise in einer Edge-Vorrichtung (300) und/oder in einer am Körper tragbaren oder portablen Vorrichtung (201) ausgeführt wird, den Computer und/oder die Rechenvorrichtung (151) und/oder die Server-Entität (400) und/oder die Edge-Vorrichtung (300) und/oder die am Körper tragbare oder portable Vorrichtung (201) veranlasst, ein Verfahren nach einem der Ansprüche 1 bis 8 durchzuführen.

12. Computerlesbares Medium, umfassend Instruktionen, die, wenn sie auf einem Computer und/oder in einer Rechenvorrichtung (151) und/oder in einer Server-Entität (400) und/oder in einer Edge-Vorrichtung (300) und/oder in einer am Körper tragbaren oder portablen Vorrichtung (201) oder teilweise in einer Rechenvorrichtung (151) und/oder teilweise in einer Server-Entität (400) und/oder teilweise in einer Edge-Vorrichtung (300) und/oder in einer am Körper tragbaren oder portablen Vorrichtung (201) ausgeführt werden, den Computer und/oder die Rechenvorrichtung (151) und/oder die Server-Entität (400) und/oder die Edge-Vorrichtung (300) und/oder die am Körper tragbare oder portable Vorrichtung (201) veranlassen, ein Verfahren nach einem der Ansprüche 1 bis 8 durchzuführen.

## Revendications

1. Procédé permettant le traçage des contacts parmi une pluralité de personnes (100) au moyen de la détection de rencontres pertinentes entre deux personnes, dans lequel chacune de la pluralité de personnes (100) porte ou transporte un dispositif à porter sur soi ou portable (201,202), les dispositifs à porter sur soi ou portables (201,202) possédant ou étant associés ou affectés respectivement à un identificateur d'étiquette (241,242), les identificateurs d'étiquette (241, 242) étant diffusés de manière répétée par les dispositifs à porter sur soi ou portables (201, 202), respectivement, à l'aide d'une interface de communication sans fil des dispositifs à porter sur soi ou portables (201, 202), dans lequel, dans une situation de contact entre un dispositif à porter sur soi ou portable spécifique (201) et un autre dispositif à porter sur soi ou portable spécifique (202), le dispositif à porter sur soi ou portable (201) est capable, au moyen de l'interface de communication sans fil du dispositif à porter sur soi ou portable (220) et par rapport à l'autre dispositif à porter sur soi ou portable (202), de déterminer de manière répétée la distance, à différents moments, par rapport à l'autre dispositif à porter sur soi ou portable (202) et de recevoir l'identificateur d'étiquette de l'autre dispositif à porter sur soi ou portable (242), générant et stockant ainsi des données de journal (261) de la situation de contact entre le dispositif à porter sur soi ou portable (201) et l'autre dispositif à porter sur soi ou portable (202),
dans lequel, afin de détecter des rencontres pertinentes entre le dispositif à porter sur soi ou portable (201) et l'autre dispositif à porter sur soi ou portable (202), le procédé comprend les étapes suivantes :
- dans une première étape, une pluralité disponible d'événements de détection de distance pertinents (272) impliquant l'autre dispositif à porter sur soi ou portable (202), détectés par le dispositif à porter sur soi ou portable (201), est déterminée au moyen de - parmi tous les événements de détection de distance disponibles (270) de l'autre dispositif à porter sur soi ou portable (202), détectés par le dispositif à porter sur soi ou portable (201) - la prise en compte ou la sélection uniquement des événements de détection de distance indiquant que l'autre dispositif à porter sur soi ou portable (202) est positionné à une distance, par rapport au dispositif à porter sur soi ou portable (201), inférieure à un seuil de distance prédéterminé (271) ou au plus égale au seuil de distance prédéterminé (271),
- dans une deuxième étape, concernant la pluralité disponible d'événements de détection de distance pertinents (272), impliquant l'autre dispositif à porter sur soi ou portable (202) et détectés par le dispositif à porter sur soi ou portable (201), un ou une pluralité d'intervalles de temps de rencontre continue (273) sont déterminés ou générés concernant des rencontres continues supposées chronologiques - d'au maximum le seuil de distance prédéterminé (271) entre le dispositif à porter sur soi ou portable (201) et l'autre dispositif à porter sur soi ou portable (202) - , dans lequel chaque intervalle de temps de rencontre continue (273) correspond à un certain nombre d'événements de détection de distance pertinents (272), chacun d'entre eux comprenant ou étant affecté à un moment respectif de l'événement de détection de distance respectif (272), la durée de l'intervalle de temps de rencontre continue étant définie ou étant déterminée pour correspondre à la différence de temps entre le plus récent et le plus précoce de ces moments, respectivement,
- dans une troisième étape, sur la base de l'intervalle ou de la pluralité d'intervalles de temps de rencontre continue détectés ou générés (273) concernant l'autre dispositif à porter sur soi ou portable (202), une durée de rencontre (274) est déterminée, la durée de rencontre (274) correspondant à la somme des durées des intervalles de temps de rencontre continue (273),
dans lequel une rencontre pertinente est détectée dans le cas où la durée de la rencontre dépasse un seuil de durée prédéterminé (275).

2. Procédé selon la revendication 1, dans lequel, concernant, au cours de la deuxième étape, la pluralité disponible d'événements de détection de distance pertinents (272), impliquant l'autre dispositif à porter sur soi ou portable (202) et détectés par le dispositif à porter sur soi ou portable (201), une rencontre chronologiquement continue est supposée avoir eu lieu et, en conséquence, un intervalle de temps de rencontre continue (273) est déterminé ou généré dans le cas où la différence de temps maximale (276) d'événements de détection de distance consécutivement détectés ou enregistrés - concernant leurs moments respectifs dans l'ordre consécutif - est inférieure ou égale à une durée d'écart maximale (277).

3. Procédé selon l'une des revendications précédentes, dans lequel la pluralité d'événements de détection de distance pertinents (272) est déterminée, notamment en prenant en compte tous les événements de détection de distance disponibles (270) liés à l'autre dispositif à porter sur soi ou portable (202), soit en continu, soit à la fin d'un intervalle de temps d'enregistrement, dans lequel l'intervalle de temps d'enregistrement correspond en particulier, ou approximativement, à l'un des éléments suivants :
- une journée ou 24 heures,
- une période de travail,
- 16 heures ou 12 heures ou 10 heures ou 9 heures ou 8 heures ou 6 heures ou 4 heures,
- 3 heures ou 2 heures ou 1 heure.

4. Procédé selon l'une des revendications précédentes, dans le cas où une rencontre pertinente est détectée, un événement de rencontre pertinent (278) est généré, dans lequel en particulier, l'événement de rencontre pertinent (278) comprend ou est lié - notamment en plus de l'identificateur d'étiquette du dispositif à porter sur soi ou portable (241) et de l'identificateur d'étiquette de l'autre dispositif à porter sur soi ou portable (242) - à au moins l'un des éléments suivants :
- tous les intervalles de temps de rencontre continue correspondants (273) liés à l'événement de rencontre pertinent (278),
- la durée de rencontre (274), dérivée de tous les intervalles de temps de rencontre continue correspondants (273) liés à l'événement de rencontre pertinent (278), ainsi que les moments respectifs les plus anciens et les plus récents de la durée totale de l'événement de rencontre pertinent (278),
- la durée de rencontre (274), dérivée de tous les intervalles de temps de rencontre continue correspondants (273) liés à l'événement de rencontre pertinent (278), ainsi que la durée totale de l'événement de rencontre pertinent (278) et une information d'horodatage indicative soit du début, soit de la fin, soit du milieu de la durée totale de l'événement de rencontre pertinent (278),
- la durée de rencontre (274), dérivée de tous les intervalles de temps de la rencontre continue correspondants (273) liés à l'événement de rencontre pertinent (278).

5. Procédé selon l'une des revendications précédentes, en tant que partie des données de journal (261) générées et stockées par le dispositif à porter sur soi ou portable (201), chacun des événements de détection de distance (270), et de même chacun des événements de détection de distance pertinents (272), détectés ou générés en ce qui concerne l'autre dispositif à porter sur soi ou portable (202), comprend un quadruple de :
- l'identificateur d'étiquette du dispositif à porter sur soi ou portable (241),
- l'identificateur d'étiquette (242) de l'autre dispositif portable ou à porter,
- une information d'horodatage indiquant le moment de l'événement de détection de distance respectif,
- une information de distance indiquant la distance détectée ou mesurée entre le dispositif à porter sur soi ou portable (201) et l'autre dispositif à porter sur soi ou portable (202),
dans lequel, en particulier, les événements de détection de distance pertinents (272) sont ceux qui, parmi tous les événements de détection de distance disponibles (270) du dispositif portable (201) ou détectés par celui-ci, ont une information de distance inférieure au seuil de distance prédéterminé (271).

6. Procédé selon l'une des revendications précédentes, dans lequel chacun des intervalles de temps de rencontre continue (273), générés à partir ou sur la base des événements de détection de distance disponibles (270) et des événements de détection de distance pertinents disponibles (272) et détectés ou générés en ce qui concerne à la fois le dispositif à porter sur soi ou portable (201) et l'autre dispositif à porter sur soi ou portable (202), comprend à la fois l'identificateur d'étiquette du dispositif à porter sur soi ou portable (241) et l'identificateur d'étiquette de l'autre dispositif à porter sur soi ou portable (242), ainsi que :
- soit une information d'horodatage indiquant le début ou la fin de l'intervalle de temps de rencontre continue respectif (273), ainsi qu'une information de durée de l'intervalle de temps de rencontre continue (273),
- soit une information d'horodatage du début de l'intervalle de temps de rencontre continue respectif (273) et une information d'horodatage de la fin de l'intervalle de temps de rencontre continue respectif (273).

7. Procédé selon l'une des revendications précédentes, dans lequel la détermination ou la sélection des événements de détection de distance pertinents (272) parmi tous les événements de détection de distance disponibles (270) est effectuée par une unité ou un module de traitement des données faisant partie du dispositif à porter sur soi ou portable (201), dans lequel les données de journal générées et stockées (261) correspondent à l'un ou à plusieurs parmi :
- tous les événements de détection de distance disponibles (270),
- tous les événements de détection de distance pertinents disponibles (272),
- les intervalles de temps de rencontre continue (273) qui y correspondent ou qui en résultent,
- les événements de rencontre pertinents (278) qui y correspondent ou qui en résultent.

8. Procédé selon l'une des revendications précédentes, dans lequel le dispositif à porter sur soi ou portable (201) est configuré pour stocker les données de journal (261) et pour transmettre les données de journal (261) à un dispositif périphérique (300) associé ou affecté au dispositif à porter sur soi ou portable (201), dans lequel la détermination ou la sélection des événements de détection de distance pertinents (272) parmi tous les événements de détection de distance disponibles (270) est effectuée par une unité ou un module de traitement de données faisant partie du dispositif périphérique (300), dans lequel les données de journal générées et stockées (261) correspondent à un ou plusieurs parmi :
- tous les événements de détection de distance disponibles (270),
- tous les événements de détection de distance pertinents disponibles (272),
- les intervalles de temps de rencontre continue (273) qui y correspondent ou qui en résultent,
- les événements de rencontre pertinents (278) qui y correspondent ou qui en résultent.

9. Système permettant le traçage des contacts entre une pluralité de personnes (100) au moyen de la détection de rencontres pertinentes entre deux personnes, dans lequel chacune de la pluralité de personnes (100) porte ou transporte un dispositif à porter sur soi ou portable (201,202), le système comprenant en particulier, outre la pluralité de dispositifs à porter sur soi ou portables (200), une entité serveur (400), un dispositif périphérique (300), et des dispositifs informatiques, les dispositifs à porter sur soi ou portables (201, 202) ayant ou étant associés ou affectés, respectivement, à un identificateur d'étiquette (241, 242), les identificateurs d'étiquette (241, 242) étant diffusés de manière répétée par les dispositifs à porter sur soi ou portables (201, 202), respectivement, à l'aide d'une interface de communication sans fil des dispositifs à porter sur soi ou portables (201, 202),
dans lequel, dans une situation de contact d'un dispositif à porter sur soi ou portable spécifique (201) avec un autre dispositif à porter sur soi ou portable spécifique (202), le dispositif à porter sur soi ou portable (201) est capable, au moyen de l'interface de communication sans fil du dispositif à porter sur soi ou portable (220) et par rapport à l'autre dispositif à porter sur soi ou portable (202), de déterminer de manière répétée la distance, à différents moments, par rapport à l'autre dispositif à porter sur soi ou portable (202) et de recevoir l'identificateur d'étiquette de l'autre dispositif à porter sur soi ou portable (242), générant et stockant ainsi des données de journal (261) de la situation de contact du dispositif à porter sur soi ou portable (201) avec l'autre dispositif à porter sur soi ou portable (202),
dans lequel, afin de détecter des rencontres pertinentes entre le dispositif à porter sur soi ou portable (201) et l'autre dispositif à porter sur soi ou portable (202), le système est configuré de telle sorte que :
- une pluralité disponible d'événements de détection de distance pertinents (272) impliquant l'autre dispositif à porter sur soi ou portable (202), détectés par le dispositif à porter sur soi ou portable (201), est déterminée au moyen de - parmi tous les événements de détection de distance disponibles (270) de l'autre dispositif à porter sur soi ou portable (202), détectés par le dispositif à porter sur soi ou portable (201) - la prise en compte ou la sélection uniquement des événements de détection de distance indiquant que l'autre dispositif à porter sur soi ou portable (202) est positionné à une distance, par rapport au dispositif à porter sur soi ou portable (201), inférieure à un seuil de distance prédéterminé (271) ou au plus égale au seuil de distance prédéterminé (271),
- concernant la pluralité disponible d'événements de détection de distance pertinents (272), impliquant l'autre dispositif à porter sur soi ou portable (202) et détectés par le dispositif à porter sur soi ou portable (201), un ou une pluralité d'intervalles de temps de rencontre continue (273) sont déterminés ou générés concernant des rencontres continues supposées chronologiques - d'au maximum le seuil de distance prédéterminé entre le dispositif à porter sur soi ou portable (201) et l'autre dispositif à porter sur soi ou portable (202) - , dans lequel chaque intervalle de temps de rencontre continue (273) correspond à un certain nombre d'événements de détection de distance pertinents (272), chacun d'entre eux comprenant ou étant affecté à un moment respectif de l'événement de détection de distance respectif (272), la durée de l'intervalle de temps de rencontre continue étant définie ou étant déterminée pour correspondre à la différence de temps entre le plus récent et le plus précoce de ces moments, respectivement,
- sur la base de l'intervalle ou de la pluralité d'intervalles de temps de rencontre continue détectés ou générés (273) concernant l'autre dispositif à porter sur soi ou portable (202), une durée de rencontre (274) est déterminée, la durée de rencontre (274) correspondant à la somme des durées des intervalles de temps de rencontre continue (273), dans lequel une rencontre pertinente est détectée si la durée de rencontre dépasse un seuil de durée prédéterminé (275).

10. Dispositifs à porter sur soi ou portables (201, 202) permettant le traçage des contacts parmi une pluralité de personnes (100) au moyen de la détection de rencontres pertinentes entre deux personnes, dans lequel chacune de la pluralité de personnes (100) porte ou transporte un dispositif à porter sur soi ou portable (201, 202) équipé d'une unité ou d'un module de traitement de données, les dispositifs à porter sur soi ou portables (201, 202) ayant ou étant associés ou affectés, respectivement, à un identificateur d'étiquette (241, 242), les identificateurs d'étiquette (241, 242) étant diffusés de manière répétée par les dispositifs à porter sur soi ou portables (201, 202), respectivement, à l'aide d'une interface de communication sans fil des dispositifs à porter sur soi ou portables (201, 202),
dans lequel, dans une situation de contact d'un dispositif à porter sur soi ou portable spécifique (201) avec un autre dispositif à porter sur soi ou portable spécifique (202), le dispositif à porter sur soi ou portable (201) est capable, au moyen de l'interface de communication sans fil du dispositif à porter sur soi ou portable (220) et par rapport à l'autre dispositif à porter sur soi ou portable (202), de déterminer de manière répétée la distance, à différents moments, par rapport à l'autre dispositif à porter sur soi ou portable (202) et de recevoir l'identificateur d'étiquette de l'autre dispositif à porter sur soi ou portable (242), générant et stockant ainsi des données de journal (261) de la situation de contact du dispositif à porter sur soi ou portable (201) avec l'autre dispositif à porter sur soi ou portable (202),
dans lequel, afin de détecter des rencontres pertinentes entre le dispositif à porter sur soi ou portable (201) et l'autre dispositif à porter sur soi ou portable (202), les dispositifs à porter sur soi ou portables (201, 202) sont configurés de telle sorte que :
- une pluralité disponible d'événements de détection de distance pertinents (272) impliquant l'autre dispositif à porter sur soi ou portable (202), détectés par le dispositif à porter sur soi ou portable (201), est déterminée par l'unité ou le module de traitement de données du dispositif à porter sur soi ou portable (201), au moyen de parmi tous les événements de détection de distance disponibles (270) de l'autre dispositif à porter sur soi ou portable (202), détectés par le dispositif à porter sur soi ou portable (201) - la prise en compte ou la sélection uniquement des événements de détection de distance indiquant que l'autre dispositif à porter sur soi ou portable (202) est positionné à une distance, par rapport au dispositif à porter sur soi ou portable (201), inférieure à un seuil de distance prédéterminé (271) ou au plus égale au seuil de distance prédéterminé (271),
- concernant la pluralité disponible d'événements de détection de distance pertinents (272), impliquant l'autre dispositif à porter sur soi ou portable (202) et détectés par le dispositif à porter sur soi ou portable (201), un ou une pluralité d'intervalles de temps de rencontre continue (273) sont déterminés ou générés concernant des rencontres continues supposées chronologiques - d'au maximum le seuil de distance prédéterminé entre le dispositif à porter sur soi ou portable (201) et l'autre dispositif à porter sur soi ou portable (202) - , dans lequel chaque intervalle de temps de rencontre continue (273) correspond à un certain nombre d'événements de détection de distance pertinents (272), chacun d'entre eux comprenant ou étant affecté à un moment respectif de l'événement de détection de distance respectif (272), la durée de l'intervalle de temps de rencontre continue étant définie ou étant déterminée pour correspondre à la différence de temps entre le plus récent et le plus précoce de ces moments, respectivement,
- sur la base de l'intervalle ou de la pluralité d'intervalles de temps de rencontre continue détectés ou générés (273) concernant l'autre dispositif à porter sur soi ou portable (202), une durée de rencontre (274) est déterminée, la durée de rencontre (274) correspondant à la somme des durées des intervalles de temps de rencontre continue (273), dans lequel une rencontre pertinente est détectée si la durée de rencontre dépasse un seuil de durée prédéterminé (275).

11. Programme comprenant un code de programme lisible par ordinateur qui, lorsqu'il est exécuté sur un ordinateur et/ou sur un dispositif informatique (151) et/ou sur une entité serveur (400) et/ou sur un dispositif périphérique (300) et/ou sur un dispositif à porter sur soi ou portable (201), ou en partie sur un dispositif informatique (151) et/ou en partie sur une entité serveur (400) et/ou en partie sur un dispositif périphérique (300) et/ou sur un dispositif à porter sur soi ou portable (201), amène l'ordinateur et/ou le dispositif informatique (151) et/ou l'entité serveur (400) et/ou le dispositif périphérique (300) et/ou le dispositif à porter sur soi ou portable (201) à effectuer un procédé selon l'une des revendications 1 à 8.

12. Support lisible par ordinateur comprenant des instructions qui, lorsqu'elles sont exécutées sur un ordinateur et/ou sur un dispositif informatique (151) et/ou sur une entité serveur (400) et/ou sur un dispositif périphérique (300) et/ou sur un dispositif à porter sur soi ou portable (201), ou en partie sur un dispositif informatique (151) et/ou en partie sur une entité serveur (400) et/ou en partie sur un dispositif périphérique (300) et/ou sur un dispositif à porter sur soi ou portable (201), amènent l'ordinateur et/ou le dispositif informatique (151) et/ou l'entité serveur (400) et/ou le dispositif périphérique (300) et/ou le dispositif à porter sur soi ou portable (201) à effectuer un procédé selon l'une des revendications 1 à 8.
